# EUROPEAN PATENT SPECIFICATION

(11) **EP 4 445 893 B1**
(45) Date of publication and mention of the grant of the patent: **21.05.2025**
(21) Application number: 23382336.8
(22) Date of filing: 11.04.2023
(51) Int. Cl.: A61K 8/92, A61K 8/73, A61Q 19/00, A61Q 19/08

(54) **SKIN CARE COMPOSITION**
HAUTPFLEGEZUSAMMENSETZUNG
COMPOSITION DE SOIN DE LA PEAU

(43) Date of publication of application: 16.10.2024
(73) Proprietor: Larrosa Díaz, María Ester, 08015 Barcelona (ES)
(72) Inventor: Larrosa Díaz, María Ester, 08015 Barcelona (ES)
(74) Representative: Isern Patentes y Marcas S.L.

(56) References cited:
- EP-A1- 3 120 831
- CN-A- 114 159 326
- CN-B- 106 109 265

## Description

### FIELD OF THE INVENTION

The present invention relates to the field of cosmetic compositions. More specifically, the invention relates to a cosmetic composition for topical use which comprises hyaluronic acid and/or sodium salts thereof and an esterified oil.

### BACKGROUND OF THE INVENTION

Skin care has become an important cause of concern in our society. Cosmetic compositions for the care of face, hands, body and/or feet are commercialized and used by numerous consumers all over the world, searching for moisturizing, reaffirming, anti-ageing, anti-wrinkle or some other effects. Many different products, in different presentation forms (serums, lotions, creams, masks...) and with a huge diversity of formulations can be found in the market, many of them exhibiting a high number of active components to produce their alleged effects, what increases their costs. And, additionally to the active ingredients, products must include supplementary ingredients such as surfactants, emulsifiers or thickening agents, which sometimes act as vehicles of the active ingredients (such as those ingredients which form micelles or liposomes encapsulating liposoluble active ingredients) or help to solubilize active ingredients which are not soluble in the vehicle of the cosmetic formulation, and which may help to obtain the desired presentation form for the product, facilitate the dispersion of other ingredients in the composition and/or confer them the desired properties of absorption, thickness, stability, preservation, etc. The number of ingredients contained in some formulations and/or the costs of some of them have often a negative influence in the final cost of the product and/or in the complexity of the manufacturing process.

In spite of the extensive research and high investments dedicated to the skin care cosmetic field, many available products do not exhibit the desired properties that make them pleasant and comfortable of use for consumers, who appreciate a feeling of smoothness or even a sort of silky sensation when touching and spreading a cosmetic product, which should be found homogeneous and easy to spread and showing a quick absorption. However, many products, particularly those in the form of serum, tonic or lotion, contain lumps and granules and are sticky and/or of slow absorption, not being pleasant for consumers even though their effects on the skin might be those desired. Transparency is also an appreciated feature in serums or tonics that not all products exhibit.

Additionally, the nature of skin poses some problems for the preparation of formulations useful to protect it, maintain a suitable water content in it and lubricate it. The primary barrier of the skin, which is very important in maintaining water balance, is the stratum corneum, which consists of dead skin cells (corneocytes) in a lipid matrix of ceramides, cholesterol and fatty acids. Suitable skin hydration is naturally maintained by the Natural Moisturizing Factor (NMF), another component of the stratum corneum primarily composed of free amino acids and derivatives of said compounds, as well as urea and lactic acid. A suitable interaction with the components of the stratum corneum the prevention of water loss and the aid to provide suitable hydration of the skin make often advisable the presence of substances of either lipophilic or hydrophilic nature often combined in the same preparations, or substances with different structural motifs where some of them are lipophilic and other ones hydrophilic. Among the moisturizers, for instance, (the ingredients aim to obtain a suitable hydration of the skin), those known as humectants are hygroscopic substances with several hydrophilic groups, which pull water from the dermis to the epidermis and stratum corneum and which can help to obtain the suitable hydration of skin by drawing water vapor from the air. Hyaluronic acid and peptides, among many others, can be considered humectants. Occlusive moisturizers, in turn, create a coating on the skin surface which diminishes and slows water loss. Petrolatum, mineral oils, lanolin, waxes, dimethicone and some other agents, usually of lipophilic nature, are common occlusive ingredients in cosmetics. But the film they form is sometimes heavy, thick and greasy to the touch, so that they are usually used in small amounts. Emolients have some similitudes with occlusive moisturizers: are not typically compatible with water, help to repair the skin and create a smooth skin surface, but by filling the spaces between dead skin cells, as their primary function is to help soften the skin. The examples of emollients include butters, oils, esters, lipids, fatty acids and ceramides.

Thus, the different natures of the possible components of the cosmetics which can be present in a same formulation, for instance different moisturizers, make advisable the presence of additional compounds, such as surfactants and emulsifiers, but also the specific properties of some specific active ingredients may pose some problems for the manufacture of many cosmetic products, while achieving the desired properties expected by consumers.

That is the case of one of the common ingredients in cosmetics, hyaluronic acid. Hyaluronic acid is a natural polysaccharide composed of a large and variable number of repeats of D-glucuronic acid and N-acetyl-D-glucosamine, whose average size can vary within a broad range of values. It is one of the chief components of the extracellular matrix with an important role, among others, in the normal epidermis, which helps to maintain a hydrated structure and even participates in wound healing and tissue regeneration. Its properties have made of hyaluronic acid, and its salts, one of the most common and well-known components of cosmetic products for skin care, due to its excellent lubricity and moisture retention properties, particularly in the case of hyaluronic acid of high molecular weight (ranging 1000-2200 kDa, particularly 1000-1800 kDa), which is the most common form added to cosmetic products; however, it is relatively glutinous, giving rise to a greasy skin feel, and practical effect is not achieved when it is added in very low amount.

In order to solve problems with hyaluronic acid, Chinese patent CN106109265B provides mixtures of three hyaluronic acid, or their salts, of high (1000 -1200 kDa, preferably 1100 kDa), medium (300 -500 kDa) and low (5 - 7 kDa) molecular weight, wherein the hyaluronic acid of high molecular weight is in a percentage of 60-62% and the other two amount, each one, to 18-20% of the total of hyaluronic acid or their salts, with preference for the relative proportions high:medium:low 6:2:2. In order to prepare the mixtures of said three hyaluronic acids, or salts thereof, they are fully ground in a mortar and mixed well. Such mixtures are presented as "raw materials" intended to be mixed with several other additional components in order to prepare commercial cosmetic products. Esters of natural oils are not mentioned among the possible additional compounds. Thus, for instance, in Test Examples 1 and 2, once the mixture of hyaluronic acids, or salts thereof, is prepared, it is used to prepare moisturizing products (an emulsion and a mask), by combining the mixture with other ingredients (water, glycerin and butanediol or pentadienol, depending on the test), so that the mixture of hyaluronic acids appears to amount to 0.5% of the final liquid formulation corresponding to said products.

According to European patent application EP3120831A1, the utility of linear hyaluronic acid as moisturizing agent is sometimes limited by the fact that it is easily degraded in the body, presenting cross-linked hyaluronic acid as a compound that improves mechanical properties and *in vivo* resistance time, while cross-linked hyaluronic acid improves mechanical properties and *in vivo* resistance. HyaCare^{®} Filler CL is mentioned as an example of commercial product comprising a water-in-oil emulsion containing small particles of cross-linked hyaluronic acid in a vehicle of water and three auxiliary agents whose function is not specified: ethylhexyl stearate, polyglyceryl-4-diisostearate/polyhydroxystearate/sebacate, and sodium isostearate. According to the manufacturer's information, that product is recommended for being added to other cosmetic products or skin care composition in a range from 0.5% to 5%, so that the auxiliary agents, whose total quantity amounts at most to 40% in HyaCare^{®} Filler CL, would amount at most to 2% in the final cosmetic product. On the other hand, as the typical administration route of hyaluronic acid, particularly cross-linked hyaluronic acid, is typically painful (because it is used as injectable filler), and the permeation of cross-lined hyaluronic acid is particularly difficult due to its size, the invention provided in EP3120831A1 aims to facilitate the delivery of hyaluronic acid, and very especially cross-linked hyaluronic acid, by topical administration. The solution provided in EP3120831A1 are low pH topical compositions, comprising a buffering agent having a pKa of about 2.8 to 4.2, and a cosmetically acceptable active ingredient, presenting as the basic exemplary composition a composition comprising five ingredients: hyaluronic acid, lactic acid, steareth 10, glycerol dilaurate and glycerin.

Some compounds with effect as emollient agents have also certain effects as surfactant and emulsifiers, but they are usually formulated together with other emulsifiers and are not so common in skin care products, particularly in serums, lotions or creams, in spite of being derivatives of natural products appreciated by their nutritional properties when ingested. That is the case of esters of natural oils, such as olive oil esters, as well as ester of other natural oils, such as those of jojoba, cocoa, macadamia seeds or babassu, which are mainly used in shampoos and other toiletries and personal hygiene products, (in general foam and cleansing products, including make-up removers), due to their dispersibility in water and the presence of a lipophilic fraction that confers them marked surfactant and cleansing properties. In the group of surfactants, they are considered non ionic surfactants. Among such esters of natural oils, some common ones are glycerine esters, PEG (polyethlyene glycol) esters and glycereth esters, that is, esters of the oil and glycereth (a polyethylene glycol ether of glycerine, which appears usually followed by a number indicating the number of ethoxylations). The recommended amount of such compounds for the preparation of formulations are around 2% for toiletries and 5% for emulsions. But, as mentioned above, compositions comprising such compounds usually include, among others, emulsifiers as well.

For instance, Korean patent KR102103140B1 discloses an external preparation for skin and a cosmetic composition comprising the same, which comprise a water-soluble ester of a natural oil, specifically an olive oil ester selected from olive oil PEG ester and olive oil glycerine ester and combinations thereof, preferably in an amount of 10% to 30% by weight, and a coconut fatty acid alkali metal salt, for use as a skin exfoliating agent. The preparation is said to dissolve keratin without any skin side effect. The ester of olive oil has the effects of imparting dispersibility in water and improving solubility in water, increasing keratin solubility. The external preparation can be included in different cosmetic formulations, such as skin lotions, lotions in the form of milks, cleansing creams and masks for eliminating keratin excess. According to the manufacturing examples, the provided formulations contain several additional ingredients, such as an emulsifier, carbomer, which is present in all of them.

Chinese patent application CN114159326A discloses cosmetic and repairing nano-compositions intended mainly for preventing and relieving skin eczema, where emulsifiers must be present and emulsification is necessary for the composition preparation. The compositions comprise necessarily DHA (the fatty acid docohexanoic acid) and other five active ingredients (ectoine, dipotassium glycirrhizinate, hyaluronic acid, squalene, vitamin E) as well as other components that are denominated auxiliary materials, emulsifiers being mentioned among them. The kind of hyaluronic acid to be used is not specified. In the compositions, the active ingredients are encapsulated in lipid vesicles formed by a phospholipid bilayer. Phospholipids, cholesterol, vitamin E polyethylene glycol succinate, emulsifiers and polyols are defined as the auxiliary material necessary to wrap DHA and the other active ingredients, improving DHA stability and reducing the fishy smell of DHA, so that the auxiliary materials, and the emulsifiers in particular, are treated as necessary ingredients. Moreover, it is preferred to mix different phospholipids, different emulsifiers and different polyols, because they are said to form a tighter, higher-strength lipid film, what increases the total number of ingredients of the compositions. Among the possible emulsifiers to be used there are two groups which appear to be esters of natural materials: one derived from a natural oil, almond oil polyglycerol-6-esters, and another one apparently derived from beeswax, polyglycerol-3-beesate, which compounds are used preferably as a mixture. Both of them are among the 14 components, additional to water, of the composition of Example 8 of application CN114159326A. The preparation of the nano-compositions of CN114159326A in general, and the composition of Example 8 in particular, requires several steps, among them one emulsifying step, the steps being: the preparation of two initial solutions (a lipid solution and an aqueous solution, each one with different ingredients), mixing both solutions, emulsifying, micronizing to obtain a micron-scale dispersion and subjecting such dispersion to nano-processing to obtain a nano-composition.

Surfactants, in turn, are chemical compounds that decrease the surface tension or interfacial tension between two liquids, a liquid and a gas, or a liquid and a solid. Those contained in cosmetics are usually organic amphiphilic compounds which contain at least one hydrophilic group and a hydrophobic group. That is the characteristic determining their compatibility with both water and oil, which allows that, when a surfactant is present in a water solution, it provokes a molecular reorganization in the solution so that the lipophilic part of the surfactant (the "hydrophobic tail") orientate itself in order to be place in the of nearby of lipid substances present in the solution, while the hydrophilic portion (the "hydrophilic tail") interacts with water, being aligned with water molecules; it can be said that the lipophilic and the hydrophilic portions of the surfactants cooperate or contribute to adsorb onto the oil/water interface. That determines the formation of structures such as micelles or the stabilization of air bubbles. Due to their capability to interact both with water molecules and compounds of lipid nature and the effects of said interaction, they are used for cleansing, foaming, thickening, emulsifying, solubilizing, penetration enhancement, antimicrobial effects, and other special effects.

The decrease in interfacial tension that surfactants provoke also has an effect in viscosity, which is usually reduced when the common surfactants used in cosmetics are added to the cosmetic formulation. Viscosity is considered an important element of cosmetic formulation. Enhancing/increasing the viscosity of aqueous surfactant systems is usually a goal to achieve in formulation development and optimisation of cosmetic products, not only for marketing reasons, but also for technical reasons: having a thickened formulation can make it possible to keep heterogeneous solutions in a stable equilibrium and simplifies the design of the suitable packaging. Additionally to enhancing the stability and overall performance of the product, thickeners impart a uniform consistency and smoothness. Therefore, the selection of the thickener is often considered a very important point in the design of cosmetic formulations.

Thickeners can be completely natural (for instance, waxes), but also synthetic or semisynthetic; they can also vary in nature, finding thickeners in the group of polysaccharides, proteins, alcohols, silicones or, as already mentioned, waxes. Although there are many available thickeners, some of them being natural-based thickeners of common use in cosmetics such as Xanthan gum, hydroxyethylcellulose or acacia gum, their use in skin care products means an additional component which increase costs and complicates the product preparation.

It would be interesting to find a formulation for a skin care product including several moisturizers, preferably including hyaluronic acid, with improved properties regarding stickiness, greasy and glutinous feels as well as easy to spread and of quick absorption. Also preferably, the product should show appropriate viscosity although having a small number of components.

The present invention provides a solution to such problem.

### DETAILED DESCRIPTION OF THE INVENTION

The present invention has been developed with the aim of improving the texture and comfort sensation experimented upon the application of a cosmetic composition comprising a hyaluronic acid or a salt thereof, where hyaluronic acid, or a salt thereof, of high molecular weight (1000 - 2200 kDa, preferably 1000 - 1800 kDa) is present and, also, hyaluronic acid, or a salt thereof, of medium (100-1000 kDa, preferably 100 - 400 kDa) and/or low (lower than 100 kDa) or preferably very low (lower than 10 kDa) molecular weights are also present. Additionally, it was also desired that the basic formulation of the preparation has the minimal ingredients which are not active ingredients as such, in order to decrease costs and facilitate the preparation process of the composition.

The solution is the cosmetic composition of the present invention, which comprises, additionally to hyaluronic acid, an ingredient that is not commonly used in formulations of facial serums or other preparations for skin care, such as creams and lotions: an ester of a natural oil. Said compounds, are discussed above, are mainly used in shampoos or other toiletries and personal hygiene products, in a recommended amount of 2% - 5% and are usually formulated in combination with compounds used as emulsifiers. Their presence in preparations where at least hyaluronic acid of high molecular weight (1500 - 1800 kDa), or a salt thereof, is present, gives rise to skin care compositions for topical use stable and transparent, where no additional emulsifier is necessary and with a suitable viscosity that also makes unnecessary the presence of a thickener, what is an interesting result since esters of natural oils have surfactant activity, as discussed above, and surfactants usually provoke a decrease of viscosity in the compositions where they have added which makes necessary the presence of a thickener in order to achieve the desired viscosity.

Thus, the resulting composition is a product with two moisturizing ingredients: hyaluronic acid (a hydrating agent which helps to provide humidity to the skin and to retain it) and the ester of a natural oil (which is an emolient which prevents humidity evaporation). Each one complements the one and are both of them compounds of natural origin. When both compounds are present, the resulting composition does not need the presence of any other compound to exhibit stability, transparency, suitable viscosity and moisturizing effect. As discussed below, other additional ingredients can be present and the presence of at least one preservative is particularly preferred.

In spite of the presence of high molecular weight hyaluronic acid (or a salt thereof, preferably the sodium salt), which is an ingredient which gives rise to a somewhat tense and uncomfortable sensation when applied, thanks to the presence of the ester of a natural oil (which softens the skin and gives rise to a certain smoothness), the composition of the present invention is well appreciated by the participants in the assays: as can be seen in Examples 1 and 2, a majority of participants preferred the serum with a formulation according to the present invention to the serum which lacked the ester of natural oil, even finding the serum according to the present invention better than the composition without any ester of a natural oil with regard to comfort sensation after being applied, absorption, hydration (moisturizing effect) and elasticity (see Example 1 below).

The above positive results were achieved thanks to the presence of the ester of a natural oil, in a proportion (5% to 15%) higher than those recommended by the providers for the preparation of toiletries and personal hygiene products.

Moreover, Example 2 shows that the product with a composition according to the invention which is an embodiment thereof where hyaluronic acid, or a salt thereof, and an ester of a natural oil are the only active ingredients and the only additional ingredient (apart from the vehicle, water) is a preservative, resulted to be a product also well appreciated by the participants in the assay, who indicated a better score for the moisturizing effect of such a product with regard to the equivalent product without the ester of a natural oil, finding the serum with the composition according to the invention also better with regard to comfort sensation after being applied, absorption and stickiness, the results with regard to absorption being similar. Such results indicate that compositions comprising an ester of a natural oil and hyaluronic acid, or a salt thereof, and preferably also a preservative, are functional and have cosmetic activity and good acceptance by the users without the need of adding other active ingredients.

Thus, the present invention relates to a cosmetic composition for topical use (that is, to be applied on the skin in one or more parts of the body, such as face, hands, feet, arms, legs or any part of the body trunk, including the ), which comprises the following components, expressed in weight/volume (w/v) percentage (grams in 100 ml) with regard to the total volume of the composition:

| | |
|---|---|
| - Hyaluronic acid of molecular weight of 1000 - 2200 kDa: or a salt thereof, | 0.1% - 1% w/v, |
| - Hyaluronic acid of molecular weight of 100 - 1000 kDa: or a salt thereof, | 0.05% - 1.5 % w/v, |
| - Hyaluronic acid of molecular weight lower than 100 kDa: or a salt thereof, | 0.05% - 1.5 % w/v, |
| - A water-soluble ester of a natural oil: | 5% - 15% w/v |
| - Water | up to the desired volume. |

The water-soluble ester can be the ester of any natural oil commonly used in toiletries, personal hygiene products (such as shampoos and other foam and cleansing products, including make-up removers) or cosmetic products, such as an ester of olive oil, almond oil, argan oil, avocado oil, babassu oil, broccoli seed oil, camelina (*Camelina sativa*) seed oil, coconut oil, cotton oil, grape seed oil, jojoba oil, linseed oil, macadamia seed oil, peach kernel oil, pomegranate seed oil, pumpkin seed oil, rice oil, safflower seed oil, sesame oil, wheat germ oil, Cannabis sativa seed oil, and mixtures thereof. The esters of olive oil are preferred.

The water-soluble ester can be in the form of a glycerine ester, polyglyceryl ester, polyethylene glycol (PEG) ester or a glycereth ester or other forms commonly used in the art. Polyglyceryl-4, polyglyceryl-6, PEG-7 and glycereth esters are preferred, which are esters available in the marked and commonly used. Among them, glycereth esters of different ethoxylation numbers, particularly glycereth-8 esters are preferred.

Olive oil glycereth-8 ester, which is the one used in the preparations of the Examples of the present application, is particularly preferred.

For the purposes of the present invention, the hyaluronic acid can be in the form of one of its salts commonly used in the art, such as the sodium or the potassium salt. The sodium salt, which is the most commonly used salt of hyaluronic acid, is preferred. Thus, for the purposes of the present invention, and as it is used in the present application, the term "hyaluronic acid" should be understood as encompassing hyaluronic acid and a salt thereof, particularly sodium hyaluronate. In the Examples of the present invention, however, where the specific formulations of the compositions assayed are indicated, the terms "hyaluronic acid" or "sodium hyaluronate" indicates the term used by the manufacturer of each specific product added to the composition.

With regard to the classification of hyaluronic acid (sodium hyaluronate) according to its average molecular weight, it is usually classified as "high", "medium" or "low" or even "very low", but the molecular weight ranges corresponding to each classification are variable depending on the information source and some classifications with additional divisions can be found. For the purposes of the present invention, hyaluronic acid (or a salt thereof, preferably sodium hyaluronate) has been classified in accordance with its average molecular weight as "high" (1000 - 2200 kDa), "medium" (100 - 1000 kDa) and "low" (lower than 100 kDa), being "very low" the one with an average molecular weight lower than 10 kDa; in the Examples, however, the products added to the composition in order to provide hyaluronic acid of each desired molecular weight have been denominated and indicated in abbreviated form following the classification, denominations and abbreviations used by the provider.

The compositions of the present invention should have hyaluronic acid (or a salt thereof) of high (1000 - 2200 kDa), medium (100 - 1000 kDa) and low (lower than 100 kDa) or very low (lower than 10 kDa) molecular weight, as expressed above. Preferably, the hyaluronic acid (or a salt thereof, preferably sodium hyaluronate) of different molecular weights will be of the range from 1000 to 1800 kDa (for high molecular weight), from 100 to 400 kDa (for medium molecular weight) and lower than 10 kDa (more preferably of the range of 1-10 kDa, or even lower than 1 kDa, which are usually hydrolyzed sodium hyaluronates) (for low molecular weight).

It is preferred that the relative proportion of hyaluronic acid (or a salt thereof) of 1000 to 1800 kDa (or 1000 to 2200 kDa) is higher than the relative proportion of the hyaluronic acid of lower molecular weights, due to its excellent lubricity and moisture retention properties. For instance, the relative proportion high : medium : low among them can be 1-7 : 0.5-1 : 0.5-2. The proportion 7:1:2 is particularly preferred, which is the one used in the preparations assayed in Examples 1 and 2. However, if an emulsifier is added to the composition (which is not necessary, but is compatible with the present invention and it is encompassed with the scope of the present invention), it would be advisable to decrease the relative proportion of the hyaluronic acid (or a salt thereof) of high molecular weight.

It is particularly preferred that the basic formulation of a composition of the present invention is the one used in Example 2 of the present invention, that is, a composition which comprises

| | |
|---|---|
| - hyaluronic acid of molecular weight of 1000 - 1800 kDa: or a salt thereof, | 0.7% w/v, |
| - hyaluronic acid of molecular weight of 100 - 400 kDa: or a salt thereof, | 0.1 % w/v, |
| - hyaluronic acid of molecular weight lower than 10 kDa: or a salt thereof, | 0.2 % w/v, |
| - olive oil glycereth-8 ester: | 10% w/v |
| - water | up to the desired volume, |

wherein the % w/v values are based on the total volume of the composition.

Other additional active ingredients (understanding as such moisturizing, filling, reaffirming, anti-cellulite, nourishing, anti-ageing, anti-wrinkling and cleansing agents, as well as other agents having effects usually alleged for cosmetic products) can also be included in the skin care preparation of the present invention, particularly when they are compounds usually present in the formulation of skin care products.

It is preferred that one or more peptides are included in the preparation, particularly one of those known as matrikines, a term that designates peptides liberated by partial proteolysis of extracellular matrix macromolecules (such as elastin, connective tissue glycoproteins or collagens), which are able to regulate cell activities being, for instance, mediators of tissue remodelling (see, for instance, Maquart et al., Critical Reviews in Oncology/Hematology, vol. 9, issue 3, March 2004, pages 199-202). Among matrikines, it is preferred that it is present:
- either the peptide known as tripeptide-1 (commonly abbreviated as GHK, because it consists of a peptide of glycine-histidine-lysine), which is a type I collagen fragment or, even more preferably, the derivative of such peptide which exhibits palmitic acid (Pal-GHK, also known as palmitoyl tripeptide-1: CAS number: 147732-56-7) attached to it, because the latter has better oil solubility, thus exhibiting better skin penetration,
   or
- the peptide known as tetrapeptide-7 (commonly abbreviated as GQPR, because it consists of a peptide of glycine-glutamine-proline-arginine) or, even more preferably, the derivative of such peptide which exhibits palmitic acid attached to it (Pal-GQPR, also known as palmitoyl tetrapeptide-7, CAS number: 221227-05-0), which is added to cosmetic products to reduce inflammation and skin damages;
   or
- mixtures thereof,
   and/or
- a mixture of the so-called "collagen peptides", which are a mixture of several peptides and collagen fragments which behaves as the natural collagen fragments present in the skin (which are involved in the natural process of reparation of the skin), provoking that lines and wrinkles are not so pronounced,
   and/or
- any other peptide or mixtures of peptides with known cosmetic effect, such as moisturizing and/or anti-ageing effects.

The peptides can be 1% to 10% w/v of the composition. If added, the % w/v of the hyaluronic acids of different molecular weights and the water-soluble ester of a natural oil should be maintained within the above indicated ranges, as in the formulations used in Example 1 when compared with those of Example 2 of the present application. The same applies if other active ingredients, or other agents with other functions (preservatives, emulsifiers, thickeners...) are added to the composition.

It is preferred that the composition of the present invention comprises, additionally to the "active ingredients" of the basic formulation set forth above, at least palmitoyl tripeptide-1, and palmitoyl tetrapeptide-7, as in Example 1 of the present application.

The peptide or mixture of peptides can be the only additional active ingredients or there can be other additional active ingredients, either supplementing the peptide effects or replacing the peptides. For instance, one or more vitamins (such as vitamin C, vitamin E, vitamin A, vitamin B3, vitamin B5, vitamin B6, vitamin F and mixtures thereof or mixtures thereof) can be present in the cosmetic composition, preferably as 5% to 20% w/v based on the total volume of the composition. Among them, the presence of vitamin C is preferred, alone or as a combination of vitamins C+E, A+C+E or C+F. Vitamin C can be added to the composition in different forms, such as ascorbic acid, ethyl ascorbic acid, ascorbyl phosphate, magnesium ascorbyl phosphate, as an ascorbyl tetraisopalmitate solution 20% in vitamin F, encapsulated, in liposomes, with cyclodextrins and other forms known for those skilled in the art. Vitamin E, in turn, can be added as tocopherol or in any other form known for those skilled in the art, alone or in combination with vitamin C. Vitamin A can be added as retinyl acetate or in any other form known for those skilled in the art, alone or in combination with vitamin C and/or vitamin E.

Preferably, if one or more vitamin/s is/are present, there will be also one or more peptides in the composition, since such complexes of active ingredients stimulate the regeneration of aged skins, reinforce their structures and contribute to a visible reduction of wrinkles.

Other active ingredients, such as ceramides, vegetal extracts, growth factors (with preference for EFG: epidermal growth factor) of plant or animal origin and/or stem cells or mixtures thereof can be also added to the composition of the present invention, either being the only additional active ingredients (necessarily additional to the hyaluronic acids and the water soluble ester of a natural oil) or in combination with one or more peptides, one or more vitamins or mixtures thereof, depending on the desired cosmetic and/or regenerating effects. Additional moisturizing agents can be also present, alone or in combination with any of the additional active ingredients previously mentioned, which moisturizing agent could be selected from the group of acetyl glucosamine, N-acetyl glucosamine (NAG), polyglutamic acid (PGA) and its sodium salts, and mixtures thereof.

The skin care composition or preparation, and any cosmetic product comprising it, can also comprise additional ingredients of common use in the cosmetic field, such as preservatives and additionally, if preferred, also one or more emulsifiers, thickeners, surfactant agents, anti-oxidant agents and/or perfumes, scents and/or aromatic or flavouring agents.

It is preferred that at least a preservative is present, such as phenoxyethanol or ethylhexylglycerin (stabilized or not with alpha-tocopherol) or mixtures thereof (commercialized as Euxyl^{®} PE9010). Such mixture is preferred due to its transparency (thus being suitable for transparent gels) and also because it is commonly used in personal care products, has a strong and broad spectrum efficacy and, besides, the presence of ethylhexylglycerin reduces the interfacial tension at the cell membrane of microorganisms and improves the antimicrobial activity. Other products with a similar compounds, such as propanediol (commercialized as Euxyl^{®} sensiva PA 40), or a combination of compounds, such as phenoxyethanol and Methylparaben and Ethylparaben (and) Propylene Glycol (commercialized as Euxyl^{®} K319) can be used.

Preferably, in order to prevent microorganism growth as best as possible, more than one preservative is present in the composition, provided that it is compatible with the safety use of the composition of the invention. The (additional) preservative may include a bacterial ferment, so that it can be a product which contains water and Leuconostoc/Radish Root Ferment Filtrate (for instance commercialized as Leucidal^{®}), which is a liquid product that prevent the growth of bacteria, yeasts and fungi in cosmetics with an aqueous phase, it is a product well tolerated by the skin, of natural origin (since it results from the fermentation of radish root by lactic bacteria of the species *Leuconostoc kimchii*) and, which contains peptides that, additionally to have antimicrobial effects, also exhibit skin hydrating properties. A *Lactobacillus* ferment (for instance, commercialized as Leucidal^{®} SF) can also be used.

Other preservatives can be also present, alone or in combination with the previous mentioned ones, such as those containing: Pentylene glycol, Levulinic acid and Glyceryl Caprylate, or Glyceryl Caprylate, or Pentylene Glycol, or Propanediol and Caprylyl Glycol and Phenylpropanol, or Pentylene Glycol and Glyceryl Caprylate, or 1,2-Hexanediol, or also those containing caprylyl glycol and Ethylhexylglycerin, or Caprylyl Glycol and Glyceryl Caprylate (and) Propanediol.

Thus, it is a preferred embodiment of the present invention a composition which consists of:

| | |
|---|---|
| a) sodium hyaluronate of molecular weight of 1000 - 1800 kDa: | 0.7% w/v, |
| b) sodium hyaluronate of molecular weight of 100 - 400 kDa: | 0.10 % w/v, |
| c) hyaluronic acid of molecular weight lower than 10 kDa: | 0.20 % w/v, |
| d) olive oil glycereth-8 ester: | 10% w/v |
| e) mixture of phenoxyethanol and ethylhexylglycerin stabilized with synthetic alpha-tocopherol | 1.0% w/v |
| f) water | 88% w/v, |

wherein the % w/v values are based on the total volume of the composition.

It is also a preferred embodiment of the present invention a composition which consists of:

| | |
|---|---|
| - sodium hyaluronate of molecular weight of 1000 - 1800 kDa: | 0.7% w/v, |
| - sodium hyaluronate of molecular weight of 100 - 400 kDa: | 0.10 % w/v, |
| - hyaluronic acid of molecular weight lower than 10 kDa: | 0.20 % w/v, |
| - olive oil glycereth-8 ester: | 10% w/v |
| Mixture of palmitoyl tripeptide-1 and palmitoyl tetrapeptide-7 mixture of phenoxyethanol and ethylhexylglycerin stabilized with synthetic alpha-tocopherol | 2% w/v, |
| | 1.0% w/v |
| - water | 86.00% w/v, |

wherein the % w/v values are based on the total volume of the composition.

On the other hand, although the formulation of the preparation of the present invention does not need the presence of emulsifiers, they can be also present, carbomer being preferred among them. One or more thickeners can be also present, if desired, although their presence is not necessary either; if added, natural-based thickeners of common use in cosmetics such as xantham gum, hydroxyethylcellulose or acacia gum or mixtures thereof are preferred.

The compositions of the present invention can be in any form useful to be applied on the skin, such a solution, a suspension, an emulsion and the like.

As used in the present application, the terms "composition" and "preparation" can be considered synonyms and equivalent. The word "formulation" is used sometimes as a synonym of said terms, but its use is preferred when the ingredients and their relative amounts are indicated therefrom.

The composition of the present invention can be the product directly used to be commercialized (after being placed in an appropriate container or mixed with the desired carrier, such as a tissue in the case of masks) or can be added to a product which will be commercialized form and/or the product applied as a cosmetic. Any cosmetic product comprising a composition of the present invention is also encompassed within the scope of the present invention. Such product can be in the form of a serum, lotion, tonic, cream, milk, gel, mask or any other suitable for topical administration onto the skin in any part of the body. The use of a composition of the present invention for the manufacture of a cosmetic product, in particular a cosmetic product of one in the already mentioned forms, is encompassed in the scope of the present invention.

Also encompassed by the scope of the present invention is the use of a composition of the present invention or a cosmetic product comprising a composition of the present invention, for skin care.

The invention will be further explained with the following Examples.

### EXAMPLES

In order to prepare the products tested in the Examples of the present application, the following ingredients were used (the supplier denominations and abbreviations are used for sodium hyaluronate /hyaluronic acid ingredients):
- Mixture of palmitoyl tripeptide-1 (Pal-GHK) and palmitoyl tetrapeptide-7 (Pal-GQPR): Matrixyl^{®} 3000 (Sesderma, Croda Iberica S.A., Barcelona, Spain)
- Medium Weight Sodium Hyaluronate (MW HA) (1000 - 1800 kDa) (Phylcare^{®} MW, Lehmann&Voss, Hamburg, Germany). (In the following Examples, the abbreviation "MW HA" will be used, in order to facilitate the association with the nomenclature used by the provider, although it can be considered that this ingredient is indeed sodium hyaluronate of high molecular weight, in accordance with the classification used in documents of the prior art such as Chinese patent CN106109265B, which is also the classification used in previous sections of the present application)
- Extra Low Weight Sodium Hyaluronate (Extra LW HA) (100 - 400 kDa) (Phylcare^{®} Extra LW, Lehmann&Voss, Hamburg, Germany)
- Extremely short-chain Hyaluronic Acid (oligo-hyaluronic acid: OL-HA or mini HA) (<10 kDa) (Phylcare^{®} OL-HA, Lehmann&Voss, Hamburg, Germany)
- Olive oil glycereth-8 ester (Resplanta Olea^{®}, ResPharma, Milano, Italy)
- Mixture of phenoxyethanol and ethylhexyglycerin stabilized with synthetic alpha-tocopherol (Euxyl^{™} PE 9010; Ashland, Barcelona, Spain)
- Leuconostoc/Radish Root Ferment Filtrate (Leucidal^{®} Liquid, Active Micro Technologies Srl, Milano, Italy)

The specific ingredients used for the preparation of each assay product and their proportion in each product are indicated in Tables 1 and 4 below, where the ingredients appear grouped as "Phase 1", "Phase 2" and "Phase 3" in accordance with the step of preparation where they are added. Thus, in order to prepare each product, the following procedure was folllowed:
a) Mixing the ingredients of Phase 1 at room temperature (or, if a quick mix is desired, at a higher temperature, preferably not higher than 40°C)
b) Hydrating the sodium hyaluronate / hyaluronic acid ingredients together with the other ingredients of Phase 2 at room temperature, until a viscous, homogeneous and transparent liquid is obtained (this phase can last from 12 to 24 hours approximately);
c) Adding the mixture of ingredients prepared in Phase 1 to the mixture of ingredients of Phase 2 and, thereafter, those of Phase 3, and mixing all of them with gently stirring, until a homogenous product is obtained.

Aliquots of 30 ml were placed in airless containers and maintain at room temperature, until they were sent to the participants in the corresponding assay.

### Example 1. Assay of the influence of the ester of natural oil: Formulation with peptides

### 1.1. Initial Assays

The assay was designed to check the influence of the presence of an ester of natural oil in the properties (mainly to check possible improvements in texture and the feeling of comfort) of a skin care product comprising a mixture of the peptides Pal-GHK and Pal-GQPR and a mixture of hyaluronic acids of high (1000 - 1800 kDa: MW HA), medium (100 - 400 kDa: Extra LW HA) and extremely low (<10kDa: OL HA) molecular weight as moisturizers, two formulations of a serum were prepared, serum 1 and serum 2, with the formulations indicated in Table 1, wherein the amount of each ingredient is indicated in weight/volume (w/v) percentage (grams per 100 ml) with regard to the total final volume of the composition:

**Table 1**

| **Ingredients** | | **Serum 1 (w/v %)** | **Serum 2 (w/v %)** |
|---|---|---|---|
| PHASE 1 | | | |
| | Mixture of peptides Pal-GHK and Pal-GQPR | 2% | 2% |
| | Distilled water | 12% | 12% |

| PHASE 2 | | | |
|---|---|---|---|
| | MW HA | 0.70% | 0.70% |
| | Extra LW HA | 0.10% | 0.10% |
| | OL-HA | 0.20% | 0.20% |
| | Preservative (mixture of phenoxyethanol and ethylhexylglycerin stabilized with synthetic alpha-tocopherol) | 1.00% | 1.00% |
| | Distilled water | 84.00% | 74.00% |

| PHASE 3 | | | |
|---|---|---|---|
| | Olive oil glycereth-8 ester | 0% | 10.00% |
| **Total** | | 100% | 100% |

Viscosity was measured with a rheometer Nahita model 810 (AuxiLab, Pamplone,Spain), using spindle number 3. Temperature: 19.8°C. Rotational speed: 12 rpm. The results were:
- serum 1: 4500 mPa·s (4500 centipoises)
- serum 2: 6500 mPa·s (6500 centipoises)

In both cases, measure error was lower than 100 mPa·s

30 ml of each serum, serum 1 and serum 2, were sent to each participant in the assay: 10 participants, most of them 40 - 60 year old, who tested each serum in a different side of the face: serum 1, left side; serum 2, right side. The serums were applied twice a day until they have been used up (30 days).

Each participant filled in a form indicating age range and skin characteristics, the serum preferred and any relevant comment about the experience, as well as a questionnaire in order to score (from 1 to 5) some characteristics, namely:
- Serum Thickness: from 1 (too thick) to 5 (optimum thickness)
- Absorption: from 1 (too slow or too quick) to 5 (optimum)
- Touch feeling and stickiness (tactile feeling): from 1 (sticky) to 5 (optimum)
- Comfort sensation after applying the product: from 1 (little comfort) to 5 (optimum comfort)
- Moisturizing/hydration: from 1 (low) to 5 (high)
- Luminosity: from 1 (low) to 5 (high)
- Wrinkles: from 1 (no amelioration) to 5 (very evident amelioration)
- Firmness: from 1 (low firmness) to 5 (high firmness)
- Elasticity: from 1 (low elasticity) to 5 (high elasticity)

The participant data and the questionnaire results are shown in Table 2 below:

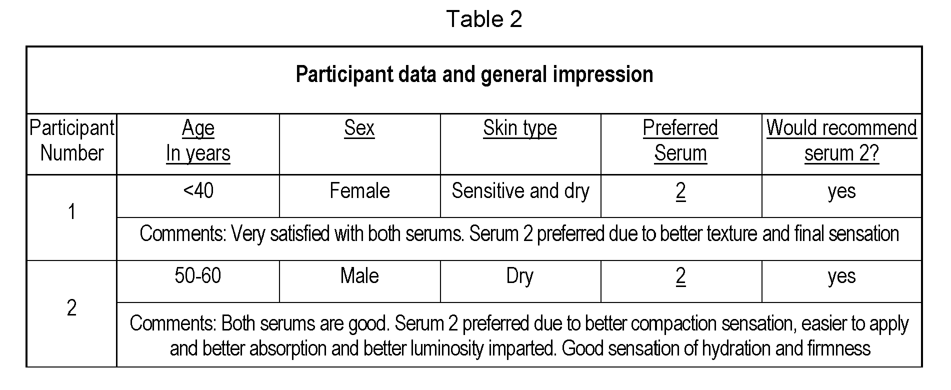

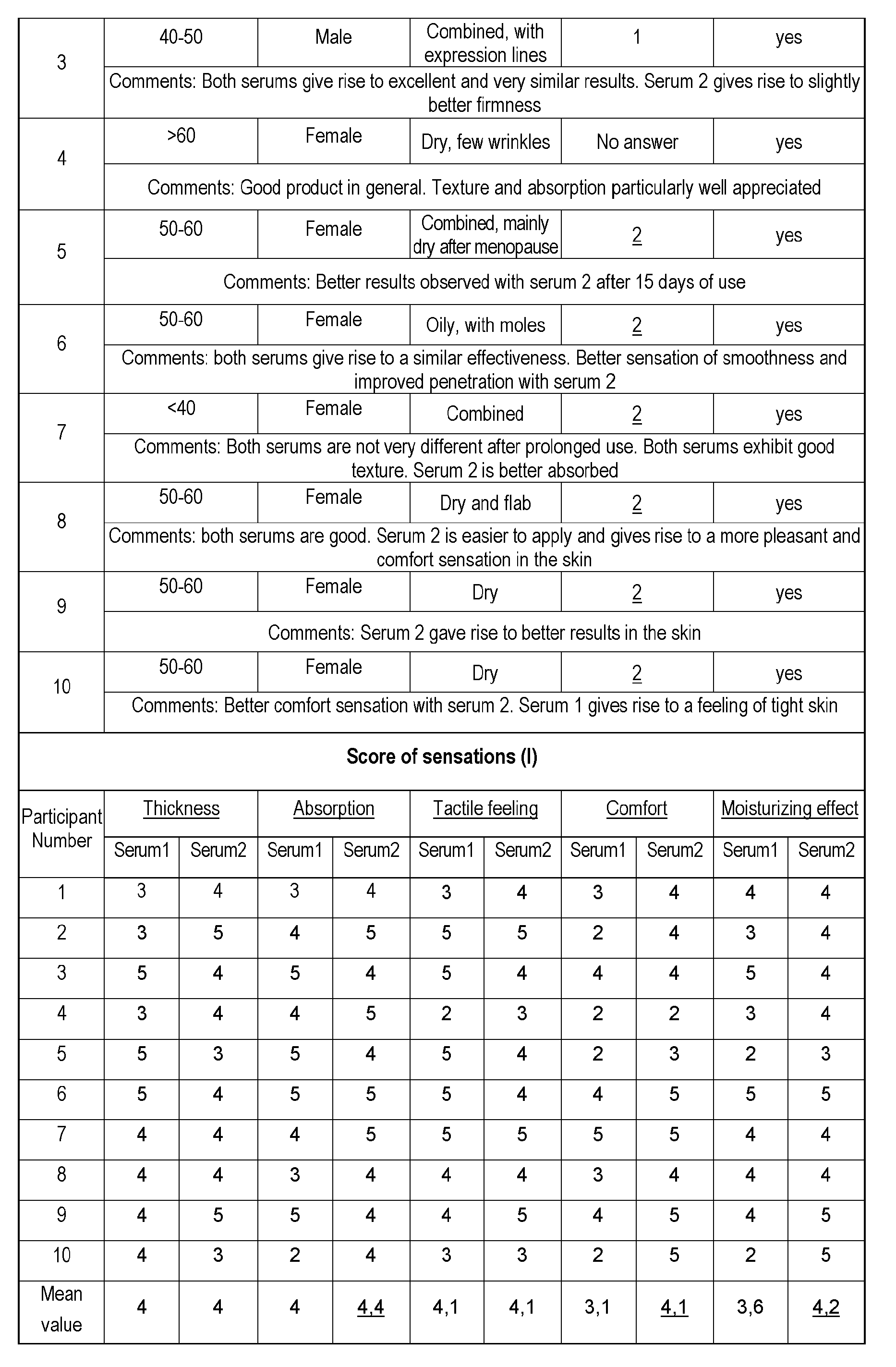

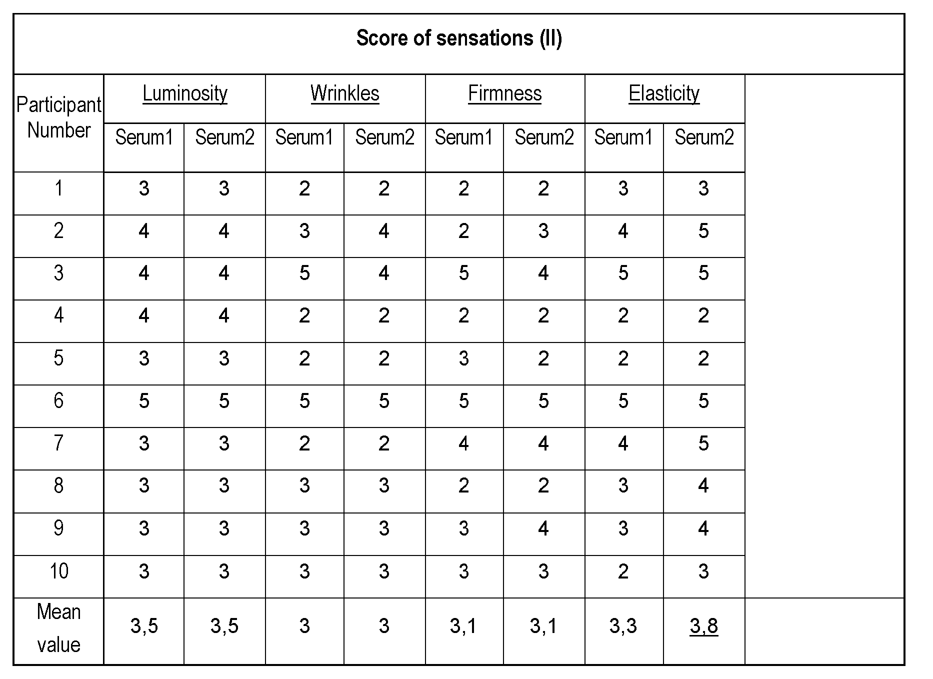

Thus, 8 participants of 10 preferred serum 2, the one with the ester of a natural oil, specifically an olive oil glycereth-8 ester. Serum 2 received a better mean score in the questions about absorption, comfort, moisturizing/hydration effect and elasticity (mean values underlined in Table 2). Such better mean scores are summarized in Table 3 below for clarity:

**Table 3**

| | Serum 2 (invention) | Serum 1 |
|---|---|---|
| Absorption | 4.4/5 | 4.1/5 |
| Comfort | 4.1/5 | 3.1/5 |
| Moisturizing/hydrating effect | 4.2/5 | 3.6/5 |
| Elasticity | 3.8/5 | 3.3/5 |

### 1.2. Serum viscosity changes with time

The serum preparations were maintained at room temperature and in the original container. Three months after the preparation of the serums, viscosity was measured again, in the same way as initially, calculating the variation with regard to the initial values. The results were:
- serum 1: 2800 mPa·s (2800 centipoises). Variation: -38%
- serum 2: 6285 mPa·s (6285 centipoises). Variation: -3%

Thus, the serum with the ester of a natural oil (serum 2) showed only a slight decrease in viscosity, while the decrease was clearly higher for the serum lacking the ester. Thus, the ester of a natural oil also has a stabilizing effect of viscosity along time that increases the shelf life of the product and improves its period of applicability in good conditions .

### Example 2. Assay of the influence of the ester of natural oil: Basic formulation

The assay was designed to check if a preparation with a basic formulation comprising only the mixture of hyaluronic acids (or salts thereof) of different molecular weights and the ester of a natural oil, supplemented with a preservative to avoid undesired microorganism growth, but without the addition of other active ingredients, were enough to obtain cosmetics effects such as those assessed in Example 1 and to receive a good score from the users, but still being preferred or improving some properties (mainly possible improvements in texture and the feeling of comfort) with regard to a similar preparation lacking the ester of a natural oil.

To that aim, two additional compositions in the form of serum were prepared, serum 3 and serum 4, with the formulations indicated in Table 4, wherein the amount of each ingredient is indicated in weight/volume (w/v) percentage (grams per 100 ml) with regard to the total final volume of the composition prepared:

**Table 4**

| **Ingredients** | | **Serum 3 (w/v %)** | **Serum 4 (w/v %)** |
|---|---|---|---|
| PHASE 1 | | | |
| | Mixture of peptides Pal-GHK and Pal-GQPR | 0% | 0% |
| | Distilled water | 9.50% | 9.50% |
| | Distilled water | 2.50% | 2.50% |

| PHASE 2 | | | |
|---|---|---|---|
| | MW HA | 0.70% | 0.70% |
| | Extra LW HA | 0.10% | 0.10% |
| | OL-HA | 0.20% | 0.20% |
| | Preservative (mixture of phenoxyethanol and ethylhexylglycerin stabilized with synthetic alpha-tocopherol) | 1.00% | 1.00% |
| | Distilled water | 86.00% | 76.00% |
| PHASE 3 | | | |
| | Olive oil glycereth-8 ester | 0% | 10.00% |
| **Total** | | 100% | 100% |

Viscosity was measured as in Example 1, obtaining similar values, the higher value corresponding to the serum with ester:
- serum 3: 4420 mPa·s (4420 centipoises)
- serum 4: 6565 mPa·s (6565 centipoises)

Analogously to the assay of Example 1, 30 ml of each serum, serum 3 and serum 4, were sent to each participant in the assay: 5 participants, all of them 40 - 60 year old, who tested each serum in a different side of the face: serum 3, left side; serum 4, right side. The serums were applied twice a day until they have been used up (30 days).

Each participant filled in a questionnaire similar to the one used for the assay of Example 1, with an analogous scale of scoring but expressing their preferences with regard to serum 3 or 4 and limiting the effects to score to serum thickness, absorption, touch feeling and stickiness (tactile feeling), comfort sensation after applying the product and moisturizing/hydration sensation. The participant data and the questionnaire results are shown in Table 5 below:

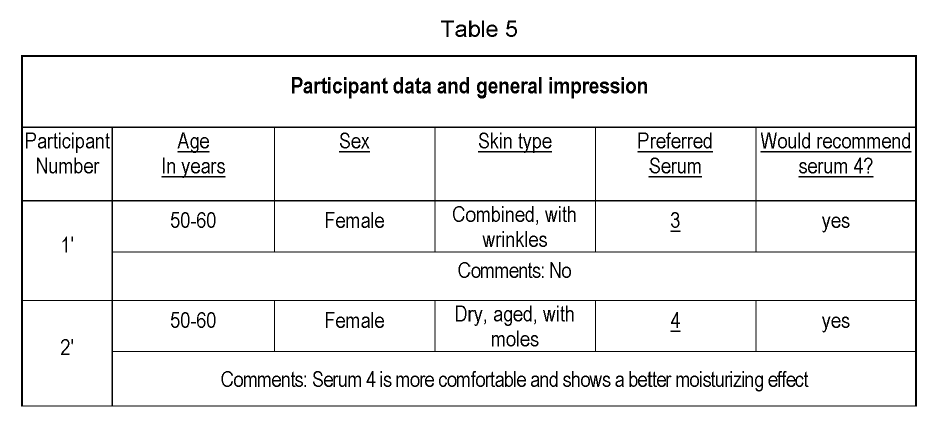

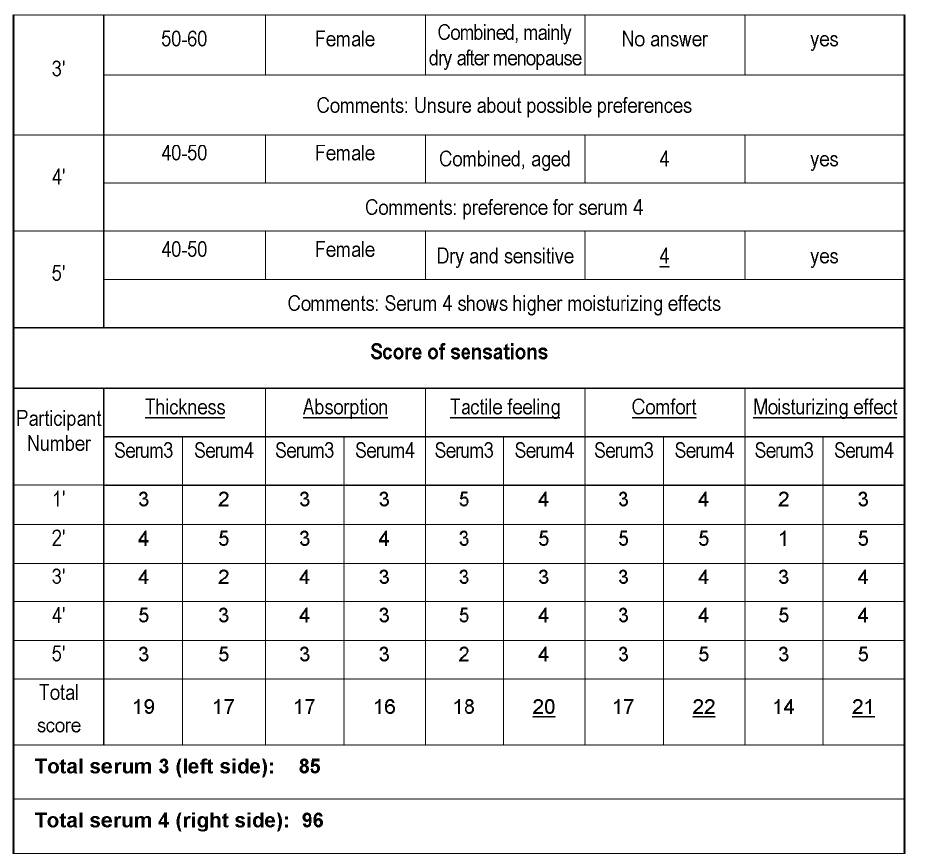

Thus, 3 participants of 5 preferred serum 4 (the one with the ester of a natural oil, specifically an olive oil glycereth-8 ester), while 1 participant were unsure about which one to select and only 1 participant preferred serum 3. Serum 2 received a better mean score in the questions about comfort, touch feeling and stickiness (tactile feeling) and moisturizing/hydration effect (specific total scores underlined in Table 5). Moreover, when the total scores received for each effect were added, the final total result was clearly higher for serum 4.

Thus, it can be concluded that the composition of the invention fulfils the desired conditions and that the embodiments where the only cosmetic active ingredients are the mixture of hyaluronic acids of different molecular weight and an ester of a natural oil, soluble in water, are enough to achieve the desired effects and to be used in a cosmetic product for skin care, as such, or being added to prepare a more complex product.

## Claims

1. A composition for topical use, **characterized in that** it comprises:
| | |
|---|---|
| - hyaluronic acid of molecular weight of 1000 - 2200 kDa: | 0.1% - 1% w/v, or a salt thereof, |
| - hyaluronic acid of molecular weight of 100 - 1000 kDa: | 0.05% - 1.5 % w/v, or a salt thereof, |
| - hyaluronic acid of molecular weight lower than 100 kDa: | 0.05% - 1.5 % w/v, or a salt thereof, |
| - a water-soluble ester of a natural oil: | 5% - 15% w/v |
| - water | up to the desired volume, |
wherein the % w/v values are based on the total volume of the composition.

2. The composition according to claim 1, wherein the water-soluble ester of a natural oil is selected from the group of an ester of olive oil, almond oil, argan oil, avocado oil, babassu oil, broccoli seed oil, camelina seed oil, coconut oil, cotton oil, grape seed oil, jojoba oil, linseed oil, macadamia seed oil, peach kernel oil, pomegranate seed oil, pumpkin seed oil, rice oil, safflower seed oil, sesame oil, shea butter, wheat germ oil, *Cannabis sativa* seed oil, and mixtures thereof.

3. The composition according to any one of the preceding claims, wherein the water-soluble ester of a natural oil is a glycerine ester, polyglyceryl ester, polyethylene glycol (PEG) ester or a glycereth ester.

4. The composition according to any one of the preceding claims, wherein the water-soluble ester of a natural oil is olive oil glycereth-8 ester.

5. The composition according to any one of the preceding claims, which comprises
| | |
|---|---|
| - hyaluronic acid of molecular weight of 1000 - 1800 kDa: | 0.1% - 1% w/v, or a salt thereof, |
| - hyaluronic acid of molecular weight of 100 - 400 kDa: | 0.05% - 1.5 % w/v, or a salt thereof, |
| - hyaluronic acid of molecular weight lower than 10 kDa: | 0.05% - 1.5 % w/v, or a salt thereof, |
| - a water-soluble ester of a natural oil: | 5% - 15% w/v |
| - water | up to the desired volume, |
wherein the % w/v values are based on the total volume of the composition.

6. The composition according to any one of claims 1 to 4, wherein the relative proportions of hyaluronic acid (or a salt thereof) of molecular weights 1000 - 2200 kDa : 100 - 1000 kDa : < 100 kDa are 1-7 : 0.5-1 : 0.5 -2.

7. The composition according to claim 5 and 6, wherein the relative proportions of hyaluronic acid (or a salt thereof) of molecular weights 1000 - 1800 kDa : 100 - 400 kDa : < 10 kDa are 7 : 1 : 2.

8. The composition according to any of the preceding claims, which additionally comprises one or more peptides, as 1% to 10% w/v based on the total volume of the composition.

9. The composition according to claim 8, which comprises tripeptide-1, palmitoyl tripeptide-1, tetrapeptide-7, palmitoyl tetrapeptide-7, or mixtures thereof, and/or collagen peptides.

10. The composition according to any one of the preceding claims, which additionally comprises:
a. one or more vitamins selected from the group of vitamin C, vitamin E, vitamin A, vitamin B3, vitamin B5, vitamin B6, vitamin F and mixtures thereof, as 5% to 20% w/v based on the total volume of the composition;
and/or
b. ceramides, vegetal extracts, growth factors of plant or animal origin and/or stem cells, acetyl glucosamine, N-acetyl glucosamine (NAG), polyglutamic acid (PGA) and/or its sodium salt, other moisturizers or mixtures thereof;
and/or
c. one or more preservative agents and/or one or more emulsifiers, thickeners, surfactant agents, anti-oxidant agents and/or perfumes, scents and/or aromatic or flavouring agents.

11. The composition according to any one of claims 1 to 7, wherein hyaluronic acid, or a salt thereof, and a water soluble ester of a natural oil are the only active ingredients and the only additional ingredient, apart from water, is a preservative.

12. The composition according to claim 5, which consists of:
| | | |
|---|---|---|
| a) | Sodium hyaluronate of molecular weight of 1000 - 1800 kDa: | 0.7% w/v, |
| b) | Sodium hyaluronate of molecular weight of 100 - 400 kDa: | 0.10% w/v, |
| c) | Hyaluronic acid of molecular weight lower than 10 kDa: | 0.20% w/v, |
| d) | Olive oil glycereth-8 ester: | 10% w/v, |
| e) | Mixture of phenoxyethanol and ethylhexylglycerin stabilized with synthetic alpha-tocoferol | 1.0% w/v |
| f) | water | 88% w/v, |
wherein the % w/v values are based on the total volume of the composition.

13. The composition according to claim 8 or 9, which consists of:
| | | |
|---|---|---|
| a) | Sodium hyaluronate of molecular weight of 1000 - 1800 kDa: | 0.70% w/v, |
| b) | Sodium hyaluronate of molecular weight of 100 - 400 kDa: | 0.10% w/v, |
| c) | Hyaluronic acid of molecular weight lower than 10 kDa: | 0.20% w/v, |
| d) | Olive oil glycereth-8 ester: | 10% w/v, |
| e) | Mixture of palmitoyl tripeptide-1 and palmitoyl tetrapeptide-7 | 2% w/v, |
| f) | Mixture of phenoxyethanol and ethylhexylglycerinstabilized with synthetic alpha-tocoferol | 1.0% w/v |
| g) | water | 86.00% w/v, |
wherein the % w/v values are based on the total volume of the composition.

14. A cosmetic product comprising a composition of any of the preceding claims.

15. The cosmetic product according to claim 14, which is in the form of a serum, lotion, tonic, cream, milk, gel or mask.

16. Use of a composition of any one of claims 1 to 13 or a product of any one of claims 14 or 15, for skin care.

## Patentansprüche

1. Zusammensetzung zur topischen Anwendung, **dadurch gekennzeichnet, dass** sie umfasst:
| | |
|---|---|
| - Hyaluronsäure mit einem Molekulargewicht von 1000 bis 2200 kDa: | 0,1% bis 1% (w/v), oder ein Salz davon, |
| - Hyaluronsäure mit einem Molekulargewicht von 100 bis 1000 kDa: | 0,05% bis 1,5% (w/v), oder ein Salz davon, |
| - Hyaluronsäure mit einem Molekulargewicht von weniger als 100 kDa: | 0,05% bis 1,5% (w/v), oder ein Salz davon, |
| - einen wasserlöslichen Ester eines natürlichen Öls: | 5% bis 15% (w/v), |
| - Wasser | bis zum gewünschten Volumen, |
wobei die % (w/v)-Werte auf das Gesamtvolumen der Zusammensetzung bezogen sind.

2. Zusammensetzung nach Anspruch 1, wobei der wasserlösliche Ester eines natürlichen Öls aus der Gruppe bestehend aus Estern von Olivenöl, Mandelöl, Arganöl, Avocadoöl, Babassuöl, Brokkolisamenöl, Kamelinasamenöl, Kokosnussöl, Baumwollöl, Traubenkernöl, Jojobaöl, Leinsamenöl, Macadamiasamenöl, Pfirsichkernöl, Granatapfelsamenöl, Kürbiskernöl, Reisöl, Distelsamenöl, Sesamöl, Sheabutter, Weizenkeimöl, Cannabis sativa-Samenöl und deren Mischungen ausgewählt ist.

3. Zusammensetzung nach einem der vorhergehenden Ansprüche, wobei der wasserlösliche Ester eines natürlichen Öls ein Glycerinester, Polyglycerinester, Polyethylenglykol-(PEG)-Ester oder Glycereth-Ester ist.

4. Zusammensetzung nach einem der vorhergehenden Ansprüche, wobei der wasserlösliche Ester eines natürlichen Öls ein Glycereth-8-Olivenölester ist.

5. Zusammensetzung nach einem der vorhergehenden Ansprüche, umfassend:
| | |
|---|---|
| - Hyaluronsäure mit einem Molekulargewicht von 1000 bis 1800 kDa: | 0,1% bis 1% (w/v), oder ein Salz davon, |
| - Hyaluronsäure mit einem Molekulargewicht von 100 bis 400 kDa: | 0,05% bis 1,5% (w/v), oder ein Salz davon, |
| - Hyaluronsäure mit einem Molekulargewicht von weniger als 10 kDa: | 0,05% bis 1,5% (w/v), oder ein Salz davon, |
| - einen wasserlöslichen Ester eines natürlichen Öls: | 5 % bis 15 % (w/v), |
| - Wasser | bis zum gewünschten Volumen, |
wobei die % (w/v)-Werte auf das Gesamtvolumen der Zusammensetzung bezogen sind.

6. Zusammensetzung nach einem der Ansprüche 1 bis 4, wobei die relativen Anteile von Hyaluronsäure (oder eines Salzes davon) mit Molekulargewichten von 1000-2200 kDa : 100-1000 kDa : < 100 kDa 1-7 : 0,5-1 : 0,5-2 betragen.

7. Zusammensetzung nach Anspruch 5 und 6, wobei die relativen Anteile von Hyaluronsäure (oder eines Salzes davon) mit Molekulargewichten von 1000-1800 kDa : 100-400 kDa : < 10 kDa 7 : 1 : 2 betragen.

8. Zusammensetzung nach einem der vorhergehenden Ansprüche, die zusätzlich ein oder mehrere Peptide umfasst, in einer Konzentration von 1% bis 10% (w/v), bezogen auf das Gesamtvolumen der Zusammensetzung.

9. Zusammensetzung nach Anspruch 8, die Tripeptid-1, Palmitoyl-Tripeptid-1, Tetrapeptid-7, Palmitoyl-Tetrapeptid-7, oder Mischungen davon, und/oder Kollagenpeptide umfasst.

10. Zusammensetzung nach einem der vorhergehenden Ansprüche, die zusätzlich umfasst:
a. ein oder mehrere Vitamine, ausgewählt aus der Gruppe bestehend aus Vitamin C, Vitamin E, Vitamin A, Vitamin B3, Vitamin B5, Vitamin B6, Vitamin F und deren Mischungen, in einer Konzentration von 5% bis 20% (w/v), bezogen auf das Gesamtvolumen der Zusammensetzung;
und/oder
b. Ceramide, pflanzliche Extrakte, Wachstumsfaktoren pflanzlichen oder tierischen Ursprungs und/oder Stammzellen, Acetylglucosamin, N-Acetylglucosamin (NAG), Polyglutaminsäure (PGA) und/oder deren Natriumsalz, andere Feuchtigkeitsspender oder deren Mischungen;
und/oder
c. ein oder mehrere Konservierungsstoffe und/oder ein oder mehrere Emulgatoren, Verdickungsmittel, Tenside, Antioxidantien und/oder Parfums, Duftstoffe und/oder Geschmacksstoffe or Aromastoffe.

11. Zusammensetzung nach einem der Ansprüche 1 bis 7, wobei Hyaluronsäure, oder ein Salz davon, und der wasserlösliche Ester eines natürlichen Öls die einzigen Wirkstoffe sind und der einzige zusätzliche Bestandteil außer Wasser ein Konservierungsstoff ist.

12. Zusammensetzung nach Anspruch 5, bestehend aus:
| | |
|---|---|
| a) Natriumhyaluronat mit einem Molekulargewicht von 1000 bis 1800 kDa: | 0,7% (w/v), |
| b) Natriumhyaluronat mit einem Molekulargewicht von 100 bis 400 kDa: | 0,10% (w/v), |
| c) Hyaluronsäure mit einem Molekulargewicht von weniger als 10 kDa: | 0,20% (w/v), |
| d) Glycereth-8-Olivenölester: | 10% (w/v), |
| e) Mischung aus Phenoxyethanol und Ethylhexylglycerin, stabilisiert mit synthetischem Alpha-Tocopherol | 1,0% (w/v), |
| f) Wasser: | 88% (w/v), |
wobei die % (w/v)-Werte auf das Gesamtvolumen der Zusammensetzung bezogen sind.

13. Zusammensetzung nach Anspruch 8 oder 9, bestehend aus:
| | |
|---|---|
| a) Natriumhyaluronat mit einem Molekulargewicht von 1000 bis 1800 kDa: | 0,70% (w/v), |
| b) Natriumhyaluronat mit einem Molekulargewicht von 100 bis 400 kDa: | 0,10% (w/v), |
| c) Hyaluronsäure mit einem Molekulargewicht von weniger als 10 kDa: | 0,20% (w/v), |
| d) Glycereth-8-Olivenöleester: | 10% (w/v), |
| e) Mischung aus Palmitoyl-Tripeptid-1 und Palmitoyl-Tetrapeptid-7 | 2% (w/v), |
| f) Mischung aus Phenoxyethanol und Ethylhexylglycerin, stabilisiert mit synthetischem Alpha-Tocopherol | 1,0% (w/v), |
| g) Wasser: | 86,00% (w/v), |
wobei die % (w/v)-Werte auf das Gesamtvolumen der Zusammensetzung bezogen sind.

14. Kosmetisches Produkt, das eine Zusammensetzung nach einem der vorhergehenden Ansprüche umfasst.

15. Kosmetisches Produkt nach Anspruch 14, in Form eines Serums, einer Lotion, eines Tonikums, einer Creme, einer Milch, eines Gels oder einer Maske.

16. Verwendung einer Zusammensetzung nach einem der Ansprüche 1 bis 13 oder eines Produkts nach einem der Ansprüche 14 oder 15, zur Hautpflege.

## Revendications

1. Une composition à usage topique, **caractérisée en ce qu'**elle comprend :
| | |
|---|---|
| - acide hyaluronique de poids moléculaire de 1000 - 2200 kDa: ou un sel de celui-ci, | 0,1% - 1% p/v, |
| - acide hyaluronique de poids moléculaire de 100 - 1000 kDa: ou un sel de celui-ci, | 0,05% - 1,5% p/v, |
| - acide hyaluronique de poids moléculaire inférieur à 100 kDa: ou un sel de celui-ci, | 0,05% - 1,5% p/v, |
| - un ester soluble dans l'eau d'une huile naturelle : | 5% - 15% p/v, |
| - eau: | jusqu'au volume souhaité, |
les valeurs de % p/v étant basées sur le volume total de la composition.

2. La composition selon la revendication 1, dans laquelle l'ester soluble dans l'eau d'une huile naturelle est choisi parmi le groupe comprenant un ester d'huile d'olive, d'huile d'amande, d'huile d'argan, d'huile d'avocat, d'huile de babassu, d'huile de graines de brocoli, d'huile de graines de caméline, d'huile de coco, d'huile de coton, d'huile de pépins de raisin, d'huile de jojoba, d'huile de lin, d'huile de graines de macadamia, d'huile de noyau de pêche, d'huile de graines de grenade, d'huile de graines de citrouille, d'huile de riz, d'huile de graines de carthame, d'huile de sésame, de beurre de karité, d'huile de germe de blé, d'huile de graines de *Cannabis sativa,* et leurs mélanges.

3. La composition selon l'une quelconque des revendications précédentes, dans laquelle l'ester soluble dans l'eau d'une huile naturelle est un ester de glycérine, un ester de polyglycérol, un ester de polyéthylène glycol (PEG) ou un ester de glycéreth.

4. La composition selon l'une quelconque des revendications précédentes, dans laquelle l'ester soluble dans l'eau d'une huile naturelle est un ester d'huile d'olive glycéreth-8.

5. La composition selon l'une quelconque des revendications précédentes, qui comprend :
| | |
|---|---|
| - acide hyaluronique de poids moléculaire de 1000 - 1800 kDa: ou un sel de celui-ci, | 0,1% - 1% p/v, |
| - acide hyaluronique de poids moléculaire de 100 - 400 kDa: | 0,05% - 1,5% p/v, ou un sel de celui-ci, |
| - acide hyaluronique de poids moléculaire inférieur à 10 kDa: ou un sel de celui-ci, | 0,05% - 1,5% p/v, |
| - un ester soluble dans l'eau d'une huile naturelle : | 5% - 15% p/v, |
| - eau | jusqu'au volume souhaité, |
les valeurs de % p/v étant basées sur le volume total de la composition.

6. La composition selon l'une quelconque des revendications 1 à 4, dans laquelle les proportions relatives d'acide hyaluronique (ou un sel de celui-ci) de poids moléculaires 1000 - 2200 kDa : 100 - 1000 kDa : < 100 kDa sont 1-7 : 0,5-1 : 0,5-2.

7. La composition selon les revendications 5 et 6, dans laquelle les proportions relatives d'acide hyaluronique (ou un sel de celui-ci) de poids moléculaires 1000 - 1800 kDa : 100 - 400 kDa : < 10 kDa sont 7 : 1 : 2.

8. La composition selon l'une quelconque des revendications précédentes, qui comprend en outre un ou plusieurs peptides, à raison de 1 % à 10 % p/v basé sur le volume total de la composition.

9. La composition selon la revendication 8, qui comprend le tripeptide-1, le palmitoyl tripeptide-1, le tétrapeptide-7, le palmitoyl tétrapeptide-7, ou leurs mélanges, et/ou des peptides de collagène.

10. La composition selon l'une quelconque des revendications précédentes, qui comprend en outre:
a. une ou plusieurs vitamines sélectionnées parmi le groupe composé de la vitamine C, la vitamine E, la vitamine A, la vitamine B3, la vitamine B5, la vitamine B6, la vitamine F et leurs mélanges, à raison de 5% à 20% p/v basé sur le volume total de la composition;
et/ou
b. des céramides, des extraits végétaux, des facteurs de croissance d'origine végétale ou animale et/ou des cellules souches, du glucosamine acétylé, du N-acétylglucosamine (NAG), de l'acide polyglutamique (PGA) et/ou son sel de sodium, d'autres hydratants ou leurs mélanges;
et/ou
c. un ou plusieurs agents conservateurs et/ou un ou plusieurs émulsifiants, épaississants, agents tensioactifs, antioxydants et/ou parfums, arômes et/ou agents aromatiques ou aromatisants.

11. La composition selon l'une quelconque des revendications 1 à 7, dans laquelle l'acide hyaluronique, ou un sel de celui-ci, et l'ester soluble dans l'eau d'une huile naturelle sont les seuls ingrédients actifs, et le seul ingrédient additionnel, à part l'eau, est un agent conservateur.

12. La composition selon la revendication 5, qui se compose de:
| | |
|---|---|
| a) Hyaluronate de sodium de poids moléculaire de 1000 - 1800 kDa: | 0,7% p/v, |
| b) Hyaluronate de sodium de poids moléculaire de 100 - 400 kDa: | 0,10% p/v, |
| c) Acide hyaluronique de poids moléculaire inférieur à 10 kDa: | 0,20% p/v, |
| d) Ester d'huile d'olive glycéreth-8: | 10% p/v, |
| e) Mélange de phénoxyéthanol et d'éthylhexylglycérine stabilisé avec alpha-tocophérol synthétique: | 1,0% p/v, |
| f) eau: | 88% p/v, |
les valeurs de % p/v étant basées sur le volume total de la composition.

13. La composition selon la revendication 8 ou 9, qui se compose de :
| | |
|---|---|
| a) Hyaluronate de sodium de poids moléculaire de 1000 - 1800 kDa: | 0,70% p/v, |
| b) Hyaluronate de sodium de poids moléculaire de 100 - 400 kDa: | 0,10% p/v, |
| c) Acide hyaluronique de poids moléculaire inférieur à 10 kDa: | 0,20% p/v, |
| d) Ester d'huile d'olive glycéreth-8: | 10% p/v, |
| e) Mélange de palmitoyl tripeptide-1 et de palmitoyl tétrapeptide-7 | 2% p/v, |
| f) Mélange de phénoxyéthanol et d'éthylhexylglycérine stabilisé avec alpha-tocophérol synthétique | 1,0% p/v, |
| g) eau: | 86,00% p/v, |
les valeurs de % p/v étant basées sur le volume total de la composition.

14. Un produit cosmétique comprenant une composition selon l'une quelconque des revendications précédentes.

15. Le produit cosmétique selon la revendication 14, qui se présente sous forme de sérum, lotion, tonique, crème, lait, gel ou masque.

16. Utilisation d'une composition selon l'une quelconque des revendications 1 à 13 ou d'un produit selon l'une quelconque des revendications 14 ou 15, pour les soins de la peau.
